# EUROPEAN PATENT SPECIFICATION

(11) **EP 2 450 294 B1**
(45) Date of publication and mention of the grant of the patent: **01.05.2013**
(21) Application number: 11179469.9
(22) Date of filing: 31.08.2011
(51) Int. Cl.: B65D 83/00, B65D 81/32, B05C 17/005

(54) **Gel applicator**
Gelaufbringer
Applicateur de gel

(30) Priority: 13.09.2010 GB 201015226
(43) Date of publication of application: 09.05.2012
(73) Proprietor: Airpure International Limited, Bury, BL9 0PB (GB)
(72) Inventor: Keeling, Robert Paul Ronan, Bury, BL8 2LE (GB)
(74) Representative: Harrison Goddard Foote

(56) References cited:
- US-A- 3 269 613
- US-A- 6 054 157

## Description

This invention relates to an applicator device for a gel composition. It is particularly concerned with a device used for applying portions of a gel stick, incorporating disinfecting and/or deodorising substances, to a surface inside the bowl of a toilet (also known as a water closet). However, it is not limited to this and may find use in other circumstances of application of a gel composition.

Conventionally, a block of material incorporating a disinfecting and/or deodorising substance has been suspended inside a toilet bowl by means of a plastics cage for slow disintegration or dissolution over a period of time to provide a disinfecting and/or deodorising effect. More recently, a gel composition incorporating an appropriate disinfecting and/or deodorising substance has been provided in the form of a stick within a tubular body of plastics material. The composition is sufficiently viscous to adhere to the surface inside the bowl of a toilet. It is applied by a user inserting a plunger into one end of the tubular body to press against a piston and push said composition out of the other end against the surface to which it is to adhere. A desired amount of the gel composition is caused to project from said other end and is broken off by moving the hollow body sideways. The remainder of the gel stick is retained within the tubular body for use on subsequent occasions until used up. A cap is typically fitted onto the end of the tubular body from which the gel is applied, which cap is removed to allow for application and then replaced for storage. When not in use the plunger is clipped into position at the side of the tubular body by means of inter-engageable projections and recesses formed on a base of the body and/or side walling of the body adjacent its base.

An object of the present invention is to provide alternative means of storing the plunger when aforesaid type of gel container/ applicator device is not in use, and to simplify use and manufacture of the device.

US 3,669,613 discloses an applicator for a gel composition having the features of the preamble of claim 1, and which comprises an elongate hollow body having a first open end from which the composition is to be dispensed and an opposing second end, a piston slidingly mounted inside the body and a plunger adapted to be fitted into the body from the second end to push the piston along the body and thereby dispense the composition from the first end, the plunger being connected to a cap adapted for removable and replaceable fitting onto the first end of the body and the plunger also being configured relative to the cap to extend side-by-side with the hollow body when the cap is fitted on to said first end of the body. In this device the plunger is a flexible, longitudinal bar which is fitted into one end of the body to provide the piston and folded over to permit fitting of the cap, at the same time, to the other end of the body.

In accordance with the present invention an applicator for a gel composition is provided which has above noted features in common with above prior art, but is characterised in that the plunger is separate from the piston and is of a hollow form with a cross-section approximately matched to the interior of the hollow body so that it is a loose sliding fit therein to apply force to the piston.

Manufacture and use of the applicator device in accordance with the invention is simplified because there is no requirement for additional interengaging formations on the hollow body and the plunger nor for manipulation to achieve connection of these components. As the plunger is connected to the cap of the container, which cap is replaced after part of contents are dispensed, the plunger is not liable to be misplaced and is immediately to hand for use when a further portion of the gel composition is to be dispensed from the hollow body.

The plunger is preferably formed integrally with, that is to say in one piece with, the cap. However, in some embodiments the plunger may be formed separately and subsequently attached to the cap during production.

The plunger is of hollow form for reasons of material economy and minimising weight of the device commensurate with adequate strength and rigidity to fulfil its purpose.

The cross-sectional shape of the plunger preferably closely matches the internal cross-section of the hollow body. In practical embodiments the hollow body and the plunger may be of oblong shape in cross-section, preferably with rounded corners, or may be circular. However, other cross-section shapes such as substantially square or rectangular are possible.

Certain embodiments of applicator in accordance with the present invention are specifically adapted for dispensing two or more different gel compositions. The effect achieved when two compositions are applied, particularly when these are of noticeably different colouration, is of a so-called dual stripe. In an embodiment of the applicator of the invention adapted for this purpose the hollow body is divided lengthwise into two (or more) compartments by a partition with a respective piston provided in each compartment and the plunger has a slot formation configured to locate over the partition so that when the plunger is inserted into the second end of the hollow body and pressed against the respective pistons, contents of both/ all compartments are dispensed at the same time.

The invention will be described further, by way of example, with reference to the accompanying drawings, in which:
Figure 1 is a front perspective view of a practical embodiment of an applicator in accordance with the present invention;
Figure 2 is a side perspective view of the same embodiment as in figure 1;
Figure 3 is a rear perspective view of the same embodiment as in figure 1;
Figure 4 is a plan view, to an enlarged scale, of the same embodiment as in figure 1;

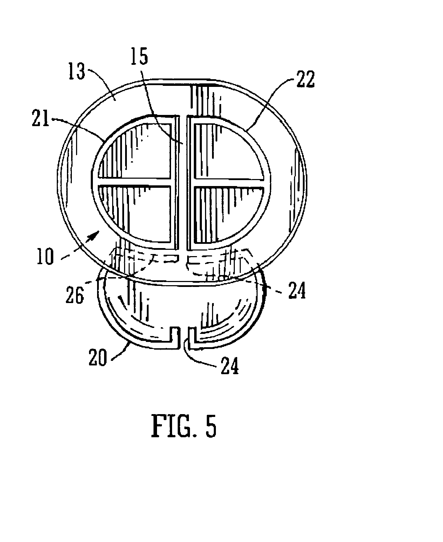

Figure 5 is an underside view of the same embodiment, also to same enlarged scale as figure 4;
Figure 6 is a perspective view, from the front of the same device as in the preceding figures, showing the device in use with cap removed and plunger inserted into one end of the body to a position where part of the contents of the body have been extruded from the other end of the body; and
Figure 7 is a perspective view corresponding to the condition shown in figure 6 but showing the device from the rear of the body.

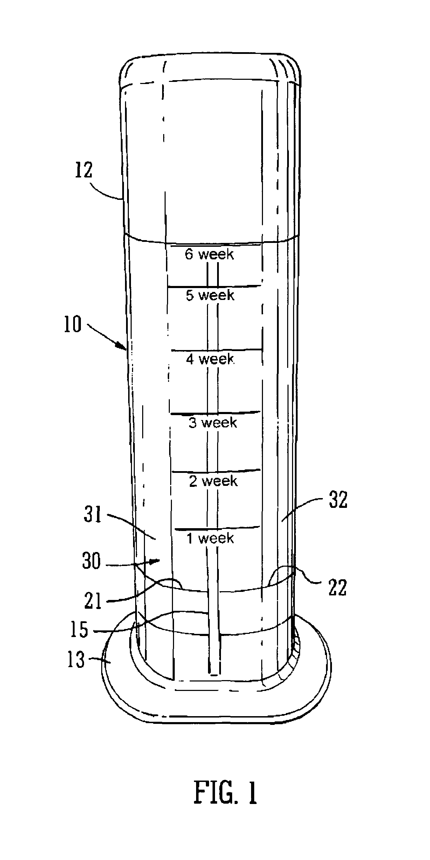

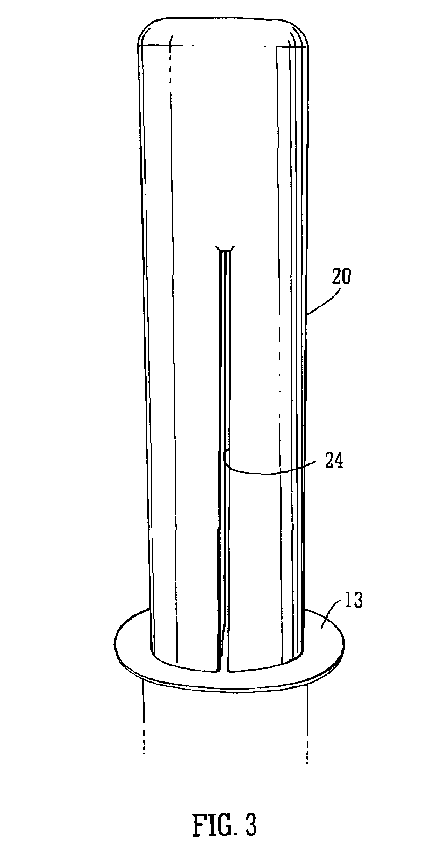

With reference to the drawings, this exemplary embodiment of applicator comprises an elongate hollow body 10 of substantially transparent plastics material filled with a gel composition 30 and an elongate hollow plunger 20 integrally formed with a cap 12 of the body 10, these being of opaque plastics material. The hollow body 10 is of oblong, approximately oval cross-section, that is to say having opposed flattened side wall sections connected by semicircularly curving end wall sections. However, the precise cross-sectional shape is not crucial to the invention and other shapes are possible in other embodiments. The body 10 has at a first end, a reduced thickness region 14 having an open end through which the gel composition 30 can be ejected. The region 14 extends from a shoulder 16 having a peripheral rebate 18 onto which the cap 12 is an interference fit, as shown in figures 1 to 3. The body 10 also has an opposing second end provided with an external flange 13 which serves as a base when the applicator is set in an upright position on a support surface, again as shown in figures 1 to 3.

Inside the body 10 a partition wall 15 is provided which subdivides the interior into two lengthwise compartments 31, 32, there being a different gel composition in each compartment. For example, one may contain a disinfecting substance, the other may contain a deodorising fragrance. There may be technical or commercial reasons for separating these. The respective gel compositions may also be of different colours. Respective pistons 21, 22 are provided which close off these compartments 31, 32 at the second end of the body, preventing passage of the gel composition 30 from that end. These pistons 21, 22 are slidably located in the interior of the body 10 and in use will be pushed upwards through the body upon insertion of the plunger 20 from the second end so as to dispense the gel composition from the reduced thickness outer region 14.

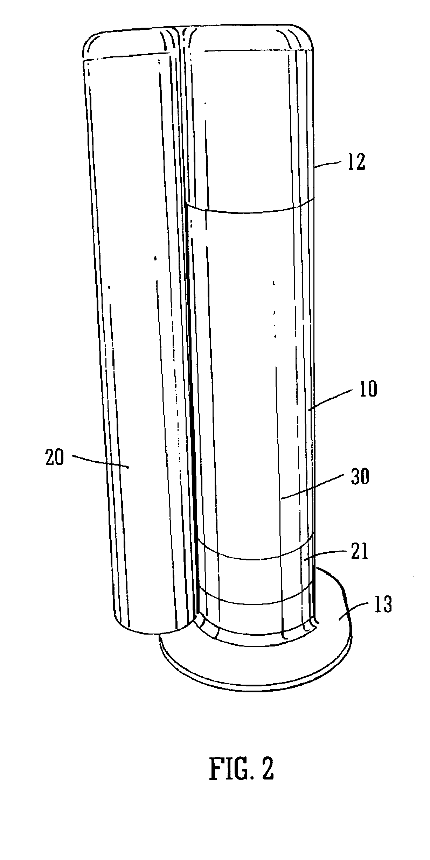

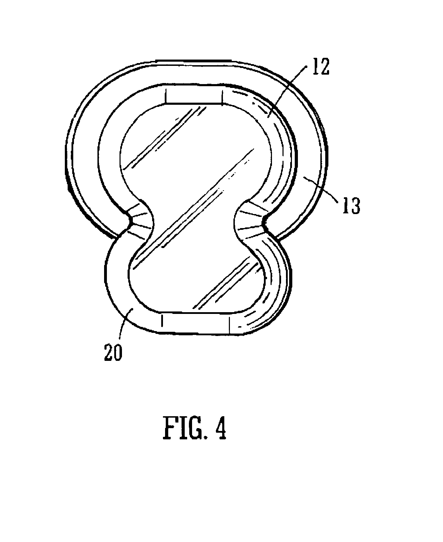

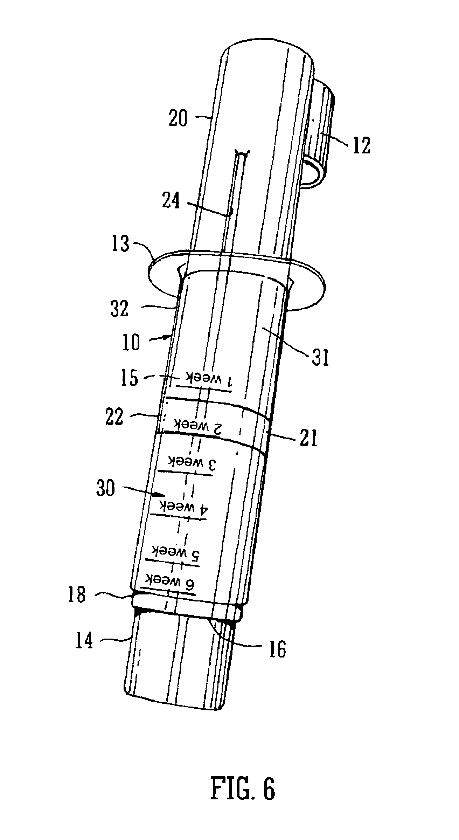

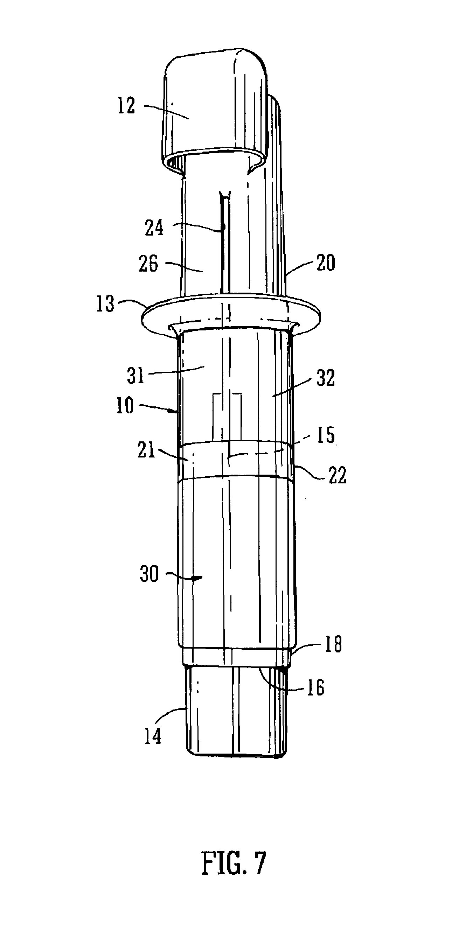

The plunger 20, as mentioned, is of hollow form. Its cross-section is approximately matched to the interior of the hollow body 10 so that it is a loose sliding fit therein and can easily be inserted into the second end of the body 10 in order to apply force to the pistons 21, 22. At opposing locations the plunger 20 is formed with elongate slots 24 to locate over the partition 15. As noted, the plunger 20 is integrally formed with the cap 12. An upper part of the plunger 20 is formed side-by-side with the cap 12, as best seen in figures 2 and 4, as well as figures 6 and 7, and the remainder of the plunger extends in alignment with said upper part. When the cap 12 is fitted onto the first end of the body 10, covering the outer region 14 and engaging onto the rebate 18, the plunger 20 extends side-by-side with the hollow body 10, as shown in figure 2. In a preferred version the plunger 20 is of such a length that it extends to just short of contact with the flange 13. The side wall of the projecting plunger nearest to the cap 12 may be formed, as in this illustrated embodiment, with a concave region 26 flanking the respective slot 24, so as to lie snugly against, i.e. in contact with, the side of the hollow body 10 when the cap 12 is fitted onto the body (figure 2).

In use, in order to apply gel composition from the hollow body, the cap 12 is removed, the free end of the plunger 20 is inserted into the flange end of the hollow body 10 and pressure is applied to the cap end of the plunger 20 to move the pistons 21, 22 to eject the required amount of gel composition from the outlet region 14. In this respect, the flange 13 is useful in providing a hand hold for a user as pressure is applied manually to the cap end of the combined plunger and cap. Thus, it may be possible to engage the flange with fingers of one hand and apply pressure to the end of the plunger with the thumb of the same hand.

When applying the gel composition to the surface of a lavatory bowl, the outlet region 14 is positioned on the surface and the first projecting part of the gel composition adheres as soon as it is ejected and makes contact with the surface. The following part of the gel composition stands proud of the surface and once the required amount has been pushed out, it can be broken off from the residue inside the body 10 by moving the body sideways. When the applicator is designed for applying gel composition to toilet bowls the body 10 typically contains sufficient gel for six weeks effective disinfectant and/or deodorising activity assuming average flushing frequency and is typically marked to indicate the amount of gel appropriate to be dispensed weekly for each successive week, as shown in figure 1.

After the required amount of gel composition has been applied, the plunger 20 is removed and the cap 12 is replaced over the outlet region 14 of the body 10 with the plunger extending adjacent the outside of the body 10 as previously.

As stated above, the foregoing description is by way of example only. The invention is not restricted to the details of the above described illustrated embodiment and may, for example, be of simpler form without subdivision of the body by a partition and without slots in the plunger. Other embodiments may be slightly more complex, including more than two compartments within the hollow body by provision of a suitable partition arrangement, appropriate positioning of slots on the plunger to accommodate these and a respective piston for each partition. In this way, several different gel compositions could be dispensed in a multi-stripe manner. Many other variations in detail, in addition to those already mentioned, are possible within the scope of the invention which is defined solely by reference to the appended claims.

## Claims

1. An applicator for a gel composition (30) comprising an elongate hollow body (10) having a first open end from which the composition is to be dispensed and an opposing second end, a piston (21, 22) slidingly mounted inside the body and a plunger (20) adapted to be fitted into the body from the second end to push the piston along the body and thereby dispense the composition from the first end, the plunger (20) being connected to a cap (12) adapted for removable and replaceable fitting onto the first end of the body and the plunger also being configured relative to the cap to extend side-by-side with the hollow body (10) when the cap is fitted on to said first end of the body, **characterised in that** the plunger (20) is separate from the piston (21, 22) and **in that** the plunger (20) is of hollow form and has a cross-section approximately matched to the interior of the hollow body (10) so that it is a loose sliding fit therein to apply force to the piston.

2. An applicator as claimed in claim 1 wherein the hollow body (10) is divided lengthwise into two compartments (31, 32) by a partition (15) with a respective piston (21, 22) in each compartment and the plunger (20) has a slot formation (24) configured to locate over the partition so that when the plunger is inserted into the second end of the hollow body and pressed against the respective pistons, contents of both compartments are dispensed at the same time.

3. An applicator as claimed in claim 1 wherein the hollow body (10) is divided lengthwise into more than two compartments by a partition with a respective piston in each compartment and the plunger has a slot formation configured to locate over the partition so that when the plunger is inserted into the second end of the hollow body and pressed against the respective pistons, contents of all the compartments are dispensed at the same time.

4. An applicator as claimed in claim 2 wherein side walling defining the hollow plunger (20) has corresponding slots (24) provided at opposing positions.

5. An applicator as claimed in any preceding claim wherein the plunger (20) is formed in one piece with the cap (12).

6. An applicator as claimed in any preceding claim wherein the hollow body (10) and the plunger (30) are each oblong in cross-section.

7. An applicator as claimed in any preceding claim wherein the body (10) is provided with an outwardly extending flange (13) at its second end and the plunger (20) has a length from its connection to the cap (12) to a free end insertable into the hollow body such that when the cap is fitted on to the first end of the body the free end of the plunger is spaced apart from the flange.

## Patentansprüche

1. Applikator für eine Gelzusammensetzung (30) mit einem länglichen hohlen Körper (10) mit einem ersten offenen Ende, aus dem die Zusammensetzung ausgegeben werden soll, und einem entgegengesetzten zweiten Ende, einem Kolben (21, 22), der gleitend innerhalb des Körpers angebracht ist, und einem Tauchkolben (20), der so ausgelegt ist, dass er vom zweiten Ende in den Körper eingefügt werden soll, um den Kolben entlang des Körpers zu schieben und dadurch die Zusammensetzung aus dem ersten Ende auszugeben, wobei der Tauchkolben (20) mit einer Kappe (12) verbunden ist, die zum abnehmbaren und austauschbaren Anfügen an das erste Ende des Körpers ausgelegt ist, und der Tauchkolben auch relativ zur Kappe so konfiguriert ist, dass er sich Seite an Seite mit dem hohlen Körper (10) erstreckt, wenn die Kappe an das erste Ende des Körpers angefügt ist, **dadurch gekennzeichnet, dass** der Tauchkolben (20) vom Kolben (21, 22) getrennt ist und dass der Tauchkolben (20) eine hohle Form aufweist und einen Querschnitt aufweist, der ungefähr an das Innere des hohlen Körpers (10) angepasst ist, so dass er sich in einem lockeren Gleitsitz darin befindet, um eine Kraft auf den Kolben aufzubringen.

2. Applikator nach Anspruch 1, wobei der hohle Körper (10) der Länge nach durch eine Trennwand (15) in zwei Abteile (31, 32) mit einem jeweiligen Kolben (21, 22) in jedem Abteil unterteilt ist, und der Tauchkolben (20) ein Schlitzgebilde (24) aufweist, das zur Anordnung über der Trennwand konfiguriert ist, so dass, wenn der Tauchkolben in das zweite Ende des hohlen Körpers eingesetzt und gegen die jeweiligen Kolben gepresst wird, die Inhalte beider Abteile gleichzeitig ausgegeben werden.

3. Applikator nach Anspruch 1, wobei der hohle Körper (10) durch eine Trennwand der Länge nach in mehr als zwei Abteile mit einem jeweiligen Kolben in jedem Abteil unterteilt ist und der Tauchkolben ein Schlitzgebilde aufweist, das zur Anordnung über der Trennwand konfiguriert ist, so dass, wenn der Tauchkolben in das zweite Ende des hohlen Körpers eingesetzt wird und gegen die jeweiligen Kolben gepresst wird, die Inhalte aller Abteile gleichzeitig ausgegeben werden.

4. Applikator nach Anspruch 2, wobei eine Seitenwand, die den hohlen Tauchkolben (20) definiert, entsprechende Schlitze (24) aufweist, die in gegenüberliegenden Positionen vorgesehen sind.

5. Applikator nach einem vorangehenden Anspruch, wobei der Tauchkolben (20) in einem Stück mit der Kappe (12) ausgebildet ist.

6. Applikator nach einem vorangehenden Anspruch, wobei der hohle Körper (10) und der Tauchkolben (30) jeweils im Querschnitt länglich sind.

7. Applikator nach einem vorangehenden Anspruch, wobei der Körper (10) mit einem sich nach außen erstreckenden Flansch (13) an seinem zweiten Ende versehen ist und der Tauchkolben (20) eine Länge von seiner Verbindung mit der Kappe (12) zu einem freien Ende, das in den hohlen Körper einsetzbar ist, aufweist, so dass, wenn die Kappe an das erste Ende des Körpers angefügt ist, das freie Ende des Tauchkolbens vom Flansch beabstandet ist.

## Revendications

1. Applicateur pour une composition de gel (30) comprenant un corps creux allongé (10) ayant une première extrémité ouverte à partir de laquelle la composition doit être distribuée et une seconde extrémité opposée, un piston (21, 22) monté de manière coulissante à l'intérieur du corps et un piston plongeur (20) adapté pour être monté dans le corps à partir de la seconde extrémité afin de pousser le piston le long du corps et distribuer ainsi la composition par la première extrémité, le piston plongeur (20) étant raccordé à un capuchon (12) adapté pour l'ajustement amovible et remplaçable sur la première extrémité du corps et le piston plongeur étant également configuré par rapport au capuchon pour s'étendre côte à côte avec le corps creux (10) lorsque le capuchon est monté sur ladite première extrémité du corps, **caractérisé en ce que** le piston plongeur (20) est séparé du piston (21, 22) et **en ce que** le piston plongeur (20) a une forme creuse et a une section transversale qui correspond approximativement à l'intérieur du corps creux (10) de sorte qu'il présente un ajustement de coulissement sans serrage à l'intérieur de ce dernier pour appliquer une force sur le piston.

2. Applicateur selon la revendication 1, dans lequel le corps creux (10) est divisé dans le sens de la longueur en deux compartiments (31, 32) par une séparation (15) avec un piston (21, 22) respectif dans chaque compartiment et le piston plongeur (20) a une formation de fente (24) configurée pour se positionner sur la séparation de sorte que le piston plongeur est inséré dans la seconde extrémité du corps creux et comprimé contre les pistons respectifs, les contenus des deux compartiments sont distribués en même temps.

3. Applicateur selon la revendication 1, dans lequel le corps creux (10) est divisé dans le sens de la longueur en plus de deux compartiments par une séparation avec un piston dans chaque compartiment et le piston plongeur a une formation de fente configurée pour se positionner sur la séparation de sorte que lorsque le piston plongeur est inséré dans la seconde extrémité du corps creux et comprimé contre les pistons respectifs, les contenus de tous les compartiments sont distribués en même temps.

4. Applicateur selon la revendication 2, dans lequel la paroi latérale définissant le piston plongeur creux (20) a des fentes (24) correspondantes prévues dans des positions opposées.

5. Applicateur selon l'une quelconque des revendications précédentes, dans lequel le piston plongeur (20) est formé d'un seul tenant avec le capuchon (12).

6. Applicateur selon l'une quelconque des revendications précédentes, dans lequel le corps creux (10) et le piston plongeur (30) sont chacun oblongs en coupe.

7. Applicateur selon l'une quelconque des revendications précédentes, dans lequel le corps (10) est prévu avec un rebord (13) s'étendant vers l'extérieur au niveau de sa seconde extrémité et le piston plongeur (20) a une longueur à partir de son raccordement au capuchon (12) jusqu'à une extrémité libre pouvant s'insérer dans le corps creux de sorte que lorsque le capuchon est monté sur la première extrémité du corps, l'extrémité libre du piston plongeur est éloignée du rebord.
